(19) Europäisches Patentamt
European Patent Office
Office européen des brevets

(11) Veröffentlichungsnummer: **0 172 379**
**B1**

(12) **EUROPÄISCHE PATENTSCHRIFT**

(45) Veröffentlichungstag der Patentschrift: **19.04.89**

(51) Int. Cl.⁴: **A 61 L 15/07**, C 08 G 18/16, C 08 G 18/10

(21) Anmeldenummer: **85108575.3**

(22) Anmeldetag: **10.07.85**

(54) **Material für Stützverbände.**

(30) Priorität: **20.07.84 DE 3426732**

(43) Veröffentlichungstag der Anmeldung: **26.02.86 Patentblatt 86/9**

(45) Bekanntmachung des Hinweises auf die Patenterteilung: **19.04.89 Patentblatt 89/16**

(84) Benannte Vertragsstaaten: **AT BE CH DE FR GB IT LI NL SE**

(56) Entgegenhaltungen:
**EP-A-0 132 675**
**GB-A-1 173 018**
**US-A-4 411 262**

(73) Patentinhaber: **BAYER AG, Konzernverwaltung RP Patentabteilung, D-5090 Leverkusen 1 Bayerwerk (DE)**

(72) Erfinder: **Richter, Roland, Dr., Roggendorfstrasse 53, D-5000 Köln 80 (DE)**
Erfinder: **Müller, Hanns Peter, Dr., Im Kerberich 6, D-5068 Odenthal (DE)**
Erfinder: **Wegner, Christian, Dr., Roggendorfstrasse 67, D-5000 Köln 80 (DE)**

**EP 0 172 379 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein verbessertes Material auf Polyurethanbasis für Stützverbände für den medizinischen oder tiermedizinischen Einsatz, welches bei Feuchtigkeitsausschluß praktisch unbegrenzt lagerstabil ist und bei Zutritt von Feuchtigkeit (z. B. Luftfeuchtigkeit oder flüssigem Wasser) rasch zu einer starren, formstabilen Struktur aushärtet.

Die Verwendung von mit Gips imprägnierten Binden als versteifendes Verbandsmaterial ist bekannt. Derartige Gipsverbände sind unerwünscht schwer, wenig luftdurchlässig, verlieren in feuchtem Zustand, beispielsweise durch Einwirkung von Wasser auf den ausgehärteten Verband, rasch an Festigkeit, sie behindern wegen ihrer Röntgenabsorption und -streuung die diagnostische Auswertung von Röntgenaufnahmen und sind wegen ihrer mangelhaften Wasserfestigkeit oft Anlaß zu Hautirritationen, hervorgerufen durch Bakterien- oder Pilzbewuchs im Verband.

Es mangelte daher nicht an Versuchen, Verbandsmaterialien zur Verfügung zu stellen, die nicht mit diesen Nachteilen behaftet sind. So wurde beispielsweise bereits versucht, Verbandsmaterial mit UV-Licht härtenden Polymerlösungen zu tränken und den damit hergestellten Verband durch Bestrahlung mit einer UV-Lampe zu härten (Chemical Orthopaedics and Related Research 103, 109-117 [1974]).

Der hierbei erforderliche Umgang mit UV-Strahlern ist umständlich; auch erreicht das UV-Licht nur die oberen Schichten des Verbands, so daß eine Härtung in tieferen Lagen gar nicht erfolgt oder längere Zeit erfordert. Ein weiterer gravierender Nachteil dieses Verfahrens ist in dem Umstand begründet, daß während des Aushärtens durch UV-Bestrahlung eine Beobachtung der Bruchstelle durch Röntgenkontrolle nicht möglich ist.

In der DT-OS-2 353 212 wird ein versteifendes Verbandsmaterial beschrieben, welches aus einem flexiblen Grundmaterial besteht, das mit Oxicarbonylisocyanatgruppen enthaltenden Substanzen ausgerüstet ist. Dieses Verbandsmaterial der DT-OS-2 353 212 konnte sich in der Praxis jedoch nicht durchsetzen, weil einerseits die Herstellung der Binden wegen der extremen Reaktivität der Oxicarbonylisocyanate auf kaum überwindbare Schwierigkeiten stieß und andererseits die Festigkeit der mit derartigen Binden hergestellten Stützverbände den Erfordernissen der Praxis nicht gerecht wurde. Außerdem führte die hohe Reaktivität der Oxicarbonylisocyanate zu Problemen bei der praktischen Anwendung, da sich die Substanzen bei Kontakt mit Wasser explosionsartig zersetzen.

In DE-OS-2 357 931 werden erstmals für die Praxis brauchbare Stützverbände angegeben, deren Härtungsprinzip die Reaktion zwischen Isocyanatgruppen und Wassermolekülen ist. Die Verbandmateralien bestehen aus einem flexiblen Trägermaterial, welches mit einer Isocyanatgruppen aufweisenden Verbindung, vorzugsweise einem Isocyanat-Präpolymer imprägniert und/oder beschichtet ist. Durch Zusatz von Katalysatoren, welche die Wasser/Isocyanat-Reaktion beschleunigen, läßt sich die Aushärtungszeit variieren. Ein Problem der Stützverbände gemäß DE-OS-2 357 931 besteht darin, daß relativ viel Katalysator zugesetzt werden muß, um die in der Praxis erwünschten kurzen Aushärtungszeiten zu erreichen. Diese hohen Katalysatorkonzentrationen führen jedoch zu einer Beeinträchtigung der Lagerstabilität der Isocyanat-Präpolymere.

Eine wesentliche Verbesserung wird gemäß DE-OS-2 651 089 dadurch erreicht, daß man als härtbare Komponente des Stützverbandes ein Präpolymer mit aromatischen NCO-Gruppen einsetzt, welches eine bestimmte Menge an Stickstoff enthält. Die Stützverbände besitzen sowohl eine befriedigende Lagerstabilität als auch die von der Praxis geforderte Aushärtung von ca. 5 - 15 Minuten.

Ein ähnlicher Lösungsweg wird in EP-A-86 621 angegeben, wonach man die Härtungsreaktion des Isocyanat-Präpolymers mit Bismorpholinodiethylether beschleunigt, d.h. einem Katalysator, der die Lagerbeständigkeit des Präpolymers wenig beeinträchtigt.

Gemäß CA-PS-1 151 960 werden als flexibles Trägermaterial bestimmte Glasfasergewebe verwendet. Die Glasfasern verleihen dem Stützverband so große Festigkeit, daß schon in einem relativ frühen Stadium der Härtungsreaktion mit Wasser weitgehende Starrheit erreicht ist. Dem Isocyanat-Präpolymer braucht daher nur relativ wenig Katalysator zugegeben zu werden, so daß die Lagerstabilität nur wenig beeinträchtigt wird. Glasfasergewebe haben jedoch für Stützverbände erhebliche Nachteile, z. B. geringe Röntgentransparenz und die Gefahr, daß an Schnittflächen die Haut des Patienten durch die starren Fasern aufgescheuert wird.

Es wurde nun gefunden, daß sich die Lagerbeständigkeit der Stützverbände erheblich verbessern und die Aushärtungszeit in weiten Grenzen einstellen läßt, wenn man in die Isocyanatgruppen aufweisende Verbindung ein Anlagerungsprodukt eines Sulfonylisocyanats an ein tert. Amin und/oder an einen Katalysator mit Zinn(II)- bzw. Zinn(IV)-carboxylat-Struktur als reversibel blockierten Katalysator einbringt. Die Katalyse der Reaktion mit Wasser läßt sich dabei überraschenderweise so einstellen, daß die Stützverbände - im Gegensatz zu jenen nach dem oben zitierten Stand der Technik - auch durch normale Luftfeuchtigkeit in weniger als 15 Minuten gehärtet werden und trotzdem sehr lagerstabil sind.

Gegenstand der Erfindung sind bei Zutritt von Feuchtigkeit härtbare Materialien für die Herstellung von Stützverbänden in der Human- oder Tiermedizin, auf Basis eines flexiblen Trägermaterials, welches mit einer Isocyanatgruppen aufweisenden Verbindung imprägniert und/oder beschichtet ist, welche dadurch gekennzeichnet sind, daß die Isocyanatgruppen aufweisende Verbindung ein Anlagerungsprodukt eines Sulfonylisocyanats an ein tert. Amin und/oder an eine Verbindung mit Zinn(II)- bzw. Zinn(IV)-carboxylat-Struktur als reversibel blockierten Katalysator enthält.

Als Trägermaterial für die erfindungsgemäßen Stützverbände kommen neben massiven oder porösen Folien

oder auch Schaumstoffen aus natürlichen oder synthetischen Materialien (z. B. Polyurethanen) in erster Linie luftdurchlässige, flexible Flächengebilde auf textiler Basis in Betracht, vorzugsweise mit einem Flächengewicht von 20 - 1000 g/$m^2$, insbesondere 30 - 500 g/$m^2$. Geeignete derartige Flächengebilde sind beispielsweise:

1. Textile Gewebe, Gewirke oder Gestricke eines Flächengewichts von 20 - 200 g/$m^2$, vorzugsweise 40 - 100 m mit einer Fadenzahl von vorzugsweise 2 - 20 Fäden je laufendem Zentimeter in der Längs- und Querrichtung. Das textile Gewebe oder Gewirke kann aus beliebigen natürlichen oder synthetischen Garnen hergestellt sein. Vorzugsweise werden jedoch Gewebe oder Gewirke eingesetzt, die aus Mischgarnen hergestellt worden sind, die ihrerseits sowohl aus hydrophoben Fäden bzw. Fasern eines hohen E-Moduls (beispielsweise Polyester) und hydrophilen natürlichen oder synthetischen Fäden bzw. Fasern (beispielsweise Baumwolle oder Polyamid) erhalten worden sind.
2. Glasfasergewebe, -gewirke oder -gestricke eines Flächengewichts von 60 bis 500 g/$m^2$, vorzugsweise 100 bis 400 g/$m^2$ und einer Fadenzahl von vorzugsweise 2 - 20 cm, in Längs- und Querrichtung. Glasfasergewebe, welche mit einer hydrophilen Schlichte versehen sind, sind bevorzugt.
3. Nicht gebundene oder gebundene oder genadelte Vliese auf Basis von anorganischen und vorzugsweise organischen Fasern eines Flächengewichts von 30 - 400 g/$m^2$, vorzugsweise 50 - 200 g/$m^2$.

Für die Herstellung erfindungsgemäßer Versteifungsverbände in Form von Schalen oder Schienen kommen auch Vliese mit Flächengewichten bis 1000 g/$m^2$ in Betracht. Erfindungsgemäß geeignete Trägermateralien werden beispielsweise auch in US-PS-4 134 397, US-PS-3 686 725, US-PS-3 882 857, DE-OS-3 211 634 und EP-A-61 642 beschrieben.

Als Isocyanatgruppen aufweisende Verbindungen kommen erfindungsgemäß alle an sich bekannten organischen Polyisocyanate in Frage, d.h. beliebige Verbindungen bzw. Gemische von Verbindungen, die pro Molekül mindestens zwei organisch gebundene Isocyanatgruppen aufweisen. Hierzu gehören sowohl niedermolekulare Polyisocyanate mit einem unter 400 liegenden Molekulargewicht als auch Modifizierungsprodukte derartiger niedermolekularer Polyisocyanate mit einem aus der Funktionalität und dem Gehalt an funktionellen Gruppen berechenbaren, z. B. 400 bis 10 000, vorzugsweise 600 bis 8 000 und insbesondere 800 bis 5 000 betragenden Molekulargewicht. Geeignete niedermolekulare Polyisocyanate sind beispielsweise solche der Formel

$$Q\,(NCO)_n,$$

in der
n = 2 - 4, vorzugsweise 2 - 3,
und
Q einen aliphatischen Kohlenwasserstoffrest mit 2 - 18, vorzugsweise 6 - 10 C-Atomen,
einen cycloaliphatischen Kohlenwasserstoffrest mit 4 - 15, vorzugsweise 5 - 10 C-Atomen,
einen aromatischen Kohlenwasserstoffrest mit 6 - 15, vorzugsweise 6 - 13 C-Atomen,
oder einen araliphatischen Kohlenwasserstoffrest mit 8 - 15, vorzugsweise 8 - 13 C-Atomen,
bedeuten.

Geeignete derartige niedermolekulare Polyisocyanate sind z. B. Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethylcyclohexan, 2,4- und 2,6-Hexahydrotoluylendiisocyanat sowie beliebige Gemische dieser Isomeren, Hexahydro-1,3- und/oder -1,4-phenylendiisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4"-triisocyanat oder Polyphenyl-polymethylenpolyisocyanate, wie sie durch Anilin-Formaldehyd-Kondensation und anschließende Phosgenierung erhalten werden.

Geeignete höhermolekulare Polyisocyanate sind Modifizierungsprodukte derartiger einfacher Polyisocyanate, d.h. Polyisocyanate mit z. B. Isocyanurat-, Carbodiimid-, Allophanat-, Biuret- oder Uretdion-Struktureinheiten, wie sie nach an sich bekannten Verfahren des Standes der Technik aus den beispielhaft genannten einfachen polyisocyanaten der oben genannten allgemeinen Formel hergestellt werden können. Unter den höhermolekularen, modifizierten Polyisocyanaten sind insbesondere die aus der Polyurethanchemie bekannten Prepolymeren mit endständigen Isocyanatgruppen des Molekulargewichtsbereichs 400 bis 10 000, vorzugsweise 600 bis 8 000 und insbesondere 800 bis 5 000 von Interesse. Diese Verbindungen werden in an sich bekannter Weise durch Umsetzung von überschüssigen Mengen an einfachen Polyisocyanaten der beispielhaft genannten Art mit organischen Verbindungen mit mindestens zwei gegenüber Isocyanatgruppen reaktionsfähigen Gruppen, insbesondere organischen Polyhydroxylverbindungen hergestellt. Geeignete derartige Polyhydroxylverbindungen sind sowohl einfache mehrwertige Alkohole wie z. B. Ethylenglykol, Trimethylolpropan, Propandiol-1,2 oder Butandiol-1,2, insbesondere jedoch höhermolekulare Polyetherpolyole und/oder Polyesterpolyole der aus der Polyurethanchemie an sich bekannten Art mit Molekulargewichten von 600 bis 8 000, vorzugsweise 800 bis 4 000, die mindestens zwei, in der Regel 2 bis 8, vorzugsweise aber 2 bis 4 primäre und/oder sekundäre Hydroxylgruppen aufweisen. Selbstverständlich können auch solche NCO-Prepolymere eingesetzt werden, die beispielsweise aus niedermolekularen Polyisocyanaten der beispielhaft

genannten Art und weniger bevorzugten Verbindungen mit gegenüber Isocyanatgruppen reaktionsfähigen Gruppen wie z. B. Polythioetherpolyolen, Hydroxylgruppen aufweisenden Polyacetalen, Polyhydroxypolycarbonaten, Hydroxylgruppen aufweisenden Polyesteramiden oder Hydroxylgruppen aufweisenden Copolymerisaten olefinisch ungesättigter Verbindungen erhalten worden sind. Zur Herstellung der NCO-Prepolymeren geeignete Verbindungen mit gegenüber Isocyanatgruppen reaktionsfähigen Gruppen, insbesondere Hydroxylgruppen, sind beispielsweise die in US-PS-4 218 543, Kolonne 7, Zeile 29 bis Kolonne 9, Zeile 25 beispielhaft offenbarten Verbindungen. Bei der Herstellung der NCO-Prepolymeren werden diese Verbindungen mit gegenüber Isocyanatgruppen reaktionsfähigen Gruppen mit einfachen Polyisocyanaten der oben beispielhaft genannten Art unter Einhaltung eines NCO/OH-Äquivalentverhältnisses von > 1 zur Umsetzung gebracht. Die NCO-Prepolymeren weisen im allgemeinen einen NCO-Gehalt von 2,5 bis 30, vorzugsweise 6 bis 25 Gew.-% auf. Hieraus geht bereits hervor, daß im Rahmen der vorliegenden Erfindung unter "NCO-Prepolymeren" bzw. unter "Prepolymeren mit endständigen Isocyanatgruppen" sowohl die Umsetzungsprodukte als solche als auch ihre Gemische mit überschüssigen Mengen an nichtumgesetzten Ausgangspolyisocyanaten, die oft auch als "Semiprepolymere" bezeichnet werden, zu verstehen sind.

Erfindungsgemäß besonders bevorzugte Polyisocyanatkomponenten sind die in der Polyurethanchemie üblichen technischen Polyisocyanate, d.h. Hexamethylendiisocyanat, 1-Isocyanato-3,3,5-trimethyl-5-isocyanatomethyl-cyclohexan (Isophorondiisocyanat, abgekürzt: IPDI), 4,4'-Diisocyanato-dicyclohexylmethan, 4,4'-Diisocyanatodiphenylmethan, dessen Gemische mit den entsprechenden 2,4'-und 2,2'-Isomeren, Polyisocyanatgemische der Diphenylmethanreihe wie sie durch Phosgenierung von Anilin/Formaldehyd-Kondensaten in an sich bekannter Weise gewonnen werden können, die Biuret- oder Isocyanuratgruppen aufweisenden Modifizierungsprodukte dieser technischen Polyisocyanate und insbesondere NCO-Prepolymere der genannten Art auf Basis dieser technischen Polyisocyanate einerseits und den beispielhaft genannten einfachen Polyolen und/oder Polyetherpolyolen und/oder Polyesterpolyolen anderseits, sowie beliebige Gemische derartiger Polyisocyanate. Isocyanate mit aromatisch gebundenen NCO-Gruppen sind erfindungsgemäß bevorzugt. Eine erfindungsgemäß besonders bevorzugte Polyisocyanat-Komponente stellt teilweise carbodiimidisiertes Diisocyanatodiphenylmethan dar, welches infolge Anlagerung von monomerem Diisocyanat an die Carbodiimid-Struktur auch Uretonimingruppen aufweist.

Erfindungsgemäß enthalten die genannten Polyisocyanate als reversibel blockierte Katalysatoren Anlagerungsprodukte (Komplexe) von Sulfonylisocyanaten an beliebige tert. Amine und/oder an Zinnkatalysatoren mit Zinn(II)- oder Zinn(IV)-Struktureinheiten.

Erfindungsgemäß geeignete Sulfonylisocyanate sind beliebige anorganische oder organische Verbindungen, die mindestens eine Struktureinheit der Formel

$$-SO_2-NCO$$

aufweisen. Vorzugsweise werden organische Sulfonylisocyanate, besonders bevorzugt solche mit aromatisch gebundenen Isocyanatosulfonylresten eingesetzt. Verfahren zur Herstellung von organischen Sulfonylisocyanaten der erfindungsgemäß geeigneten Art, sowie ihr chemisches Verhalten werden zusammenfassend dargestellt von H. Ulrich in Chem. Rev. 65, Seite 369 - 376, 1965. Des weiteren ist die Herstellung von Arylsulfonylisocyanaten beschrieben in den US-PS-2 666 787 und 3 484 466. Erfindungsgemäß können sowohl aliphatische, cycloaliphatische als auch aromatische Mono- oder Polysulfonylisocyanate eingesetzt werden. Als Beispiele seien genannt: Methylsulfonylisocyanat, Butylsulfonylisocyanat, Cyclohexylsulfonylisocyanat, Perfluoroctylsulfonylisocyanat, Phenylsulfonylisocyanat, p-Toluolsulfonylisocyanat, Benzylsulfonylisocyanat, p-Chlorphenylsulfonylisocyanat, m-Nitrophenylsulfonylisocyanat, 2,5-Dimethylphenylsulfonylisocyanat, p-Fluorphenylsulfonylisocyanat, 2,5-Dichlorphenylsulfonylisocyanat, 3,4-Dichlorphenylsulfonylisocyanat, p-Bromphenylsulfonylisocyanat, p-Methoxyphenylsulfonylisocyanat, p-Nitrophenylsulfonylisocyanat und o-Nitrophenylsulfonylisocyanat; m-Phenylendisulfonyldiisocyanat, p-Phenylendisulfonyldiisocyanat, 4-Methyl-m-phenylendisulfonyldiisocyanat, 2-Chlor-p-phenylendisulfonyldiisocyanat, 5-Chlor-m-Phenylendisulfonyldiisocyanat, 1,5-Naphthylendisulfonyldiisocyanat, 3-Nitro-p-phenylendisulfonyldiisocyanat, 4-Methoxy-m-Phenylendisulfonyldiisocyanat, 2,5-Furandiylbis - (methylensulfonyl)-diisocyanat, 4,4'-Biphenylendisulfonyldiisocyanat, 2,2'-Dichlor-4,4'-biphenylylendisulfonyldiisocyanat, 3,3'-Dimethoxy-4,4'-biphenylylendisulfonyldiisocyanat, (Methylendi-p-phenylen)disulfonyldiisocyanat, (Methylendi-3,3'-dimethoxy-p-phenylen)disulfonyldiisocyanat, (Methylendi-3,3'-dimethyl-p-phenylen)disulfonyldiisocyanat und 2-Methyl-p-phenylendisulfonyldiisocyanat; des weiteren Sulfonylisocyanate mit freien NCO-Gruppen wie m-Isocyanatophenylsulfonylisocyanat, P-Isocyanatophenylsulfonylisocyanat, 3-Isocyanato-p-tolylsulfonylisocyanat, 5-Isocyanato-o-tolylsulfonylisocyanat, 3-Isocyanato-4-methoxyphenylsulfonylisocyanat, 4-Isocyanato-3-chlorphenylsulfonylisocyanat, 4'-Isocyanato-4-biphenylylsulfonylisocyanat, 4'-Isocyanato-2,2'-dichloro-4-biphenylylsulfonylisocyanat, 4'-Isocyanato-3,3'-dimethoxy-4-biphenylsulfonylisocyanat, α-(p-Isocyanatophenyl)-p-tolylsulfonylisocyanat, α-(4-Isocyanato-3-methoxyphenyl)-2-methoxy-p-tolylsulfonylisocyanat, α-(4-Isocyanato-m-tolyl)-2,4-xylylsulfonylisocyanat und 5-Isocyanato-1-naphthylsulfonylisocyanat; oder mit freien Isothiocyanat-Gruppen wie p-Isothiocyanatophenylsulfonylisocyanat, m-Isothiocyanatophenylsulfonylisocyanat, 3-Isothiocyanato-4-methoxylphenylsulfonylisocyanat und 4-Isothiocyanato-3-methylphenylsulfonylisocyanat.

Bevorzugte Verwendung finden Sulfonylisocyanate, deren $-SO_2-NCO$-Gruppe direkt an einen aromatischen

Rest gebunden ist; ganz bevorzugt sind Phenylsulfonylisocyanat, p-Chlorphenylsulfonylisocyanat und P-Toluolsulfonylisocyanat (Tosylisocyanat). Oft empfiehlt sich auch die Verwendung von solchen Sulfonylisocyanaten der beispielhaft genannten Art, welche entweder mindestens zwei Isocyanatosulfonyl-Struktureinheiten oder neben einer Isocyanatosulfonyl-Struktureinheit weitere Isocyanatgruppen aufweisen, da derartige polyfunktionelle Verbindungen bei der Aushärtung mit Wasser in das resultierende Polyurethangerüst ohne Kettenabbruch eingebaut werden.

Neben den beispielhaft genannten organischen Sulfonylisocyanaten kommen erfindungsgemäß auch anorganische Sulfonylisocyanate wie z. B. Chlorsulfonylisocyanat oder Sulfonyldiisocyanat, deren Herstellung beispielsweise in DE-PS-928 896 bzw. in DE-PS-1 152 023 beschrieben ist, in Betracht. Geeignet sind auch Oxysulfonylisocyanate wie z. B. Trimethylsilyloxysulfonylisocyanat.

Für die Herstellung der reversibel blockierten Katalysatoren geeignete tert. Amine bzw. Zinnverbindungen sind alle beliebigen aus der Polyurethanchemie an sich bekannten Katalysatoren, die entweder mindestens eine tert. Aminogruppe oder mindestens eine Zinn(II)- bzw. Zinn(IV)-Carboxylat-Struktureinheit aufweisen. Vorzugsweise werden solche tert. Amine bzw. Zinncarboxylate eingesetzt, die keine mit Isocyanatgruppen reaktionsfähigen Gruppen, insbesondere keine Hydroxyl- oder primäre bzw. sekundäre Aminogruppe aufweisen, da derartige Reaktivgruppen mit dem Sulfonylisocyanat zu Urethan oder Harnstoff abreagieren und dann erst mit weiterem Sulfonylisocyanat durch Umsetzung mit dem tert. Stickstoff bzw. dem Carboxylatanion die erfindungswesentlichen Addukte bilden können, die dann bei Zutritt von Wasser nicht mehr den ursprünglichen Katalysator freisetzen, sondern das entsprechend derivatisierte Produkt. Da dieses aber durchaus noch katalytische Wirkung aufweisen kann, ist die Verwendung von Katalysatoren, die neben einem tert. Stickstoffatom bzw., neben der Zinncarboxylat-Struktur noch freie OH- oder aminische NH-Gruppen aufweisen, zwar weniger bevorzugt, jedoch auch nicht ausgeschlossen. Im übrigen können die erfindungsgemäß geeigneten tert. Amine bzw. Zinnverbindungen selbstverständlich beliebige, unter den Bedingungen der Härtungsreaktion mit Wasser inerte, und die erfindungsgemäße Verwendung der Verbandmaterialien nicht störende, Heteroatome enthaltende Struktureinheiten aufweisen.

Die erfindungsgemäß geeigneten tert. Amine weisen im allgemeinen ein zwischen 101 und 600, vorzugsweise 101 bis 300 liegendes Molekulargewicht auf. Beispiele geeigneter tert. Amine sind Triethylamin, Tributylamin, N-Methyl-morpholin, N-Ethyl-morpholin, N,N,N',N'-Tetramethyl-ethylendiamin, Pentamethyl-diethylentriamin und höhere Homologe (DE-OS-2 624 527 und DE-OS-2 624 528), 1,4-Diazabicyclo-(2,2,2)-octan, N-Methyl-N'-dimethylaminoethylpiperazin, Bis-(dimethylaminoalkyl)-piperazine (DE-OS-2 636 787), N,N-Dimethylbenzylamin, N,N-Dimethylcyclohexylamin, N,N-Diethylbenzylamin, Bis-(N,N-diethylaminoethyl)-adipat, N,N,N',N'-Tetramethyl-1,3-butandiamin, N,N-Dimethyl-β-phenylethylamin, 1,2-Dimethylimidazol, 2-Methylimidazol, monocyclische und bicyclische Amidine (DE-OS-1 720 633), Bis-(dialkylamino)alkyl-ether (US-PS-3 330 782, DE-AS-1 030 558, DE-OS-1 804 361 und DE-OS-2 618 280), Amidgruppen (vorzugsweise Formamidgruppen) aufweisende tert. Amine, gemäß der DE-OS-2 523 633 und DE-OS-2 732 292), Pyridin, Aminopyridine wie 4-Dimethylaminopyridin, N,N',N''-Tris(dimethylaminopropyl)-s-hexahydrotriazin, N,N-Diethylcyclohexylamin, N,N,N',N'-Tetramethylmethandiamin, 2,2'-Sulfobis(H,N-dimethylethylamin), Bis[2(3'-N,N-dimethylaminopropoxy)-ethyl]ether, und N-(3-Dimethylaminopropyl)-morpholin. Ferner seien genannt Silaamine mit Kohlenstoff-Silicium-Bindungen, wie sie z. B. in der DE-PS-1 229 290 beschrieben sind, z. B. 2,2,4-Trimethyl-2-silamorpholin und 1,3-Diethylaminomethyl-tetramethyl-disiloxan. Erfindungsgemäß können jedoch auch die tertiären Aminstickstoffatome in der Polyisocyanat-Komponente chemisch eingebaut sein. Verbindungen dieses Typs erhält man z. B., indem man Polyisocyanate mit tert. Aminstickstoff aufweisenden Alkoholen oder anderen Verbindungen mit reaktivem Wasserstoff umsetzt, z. B. mit Dimethylethanolamin, Dibutylethanolamin oder den in DE-OS-2 651 089 beschriebenen Aminopolyolen.

Als erfindungsgemäß bevorzugte organische Zinnsalze seien beispielsweise genannt:

Zinn(II)-salze von Carbonsäuren wie Zinn(II)-acetat, Zinn(II)-octoat, Zinn(II)-ethylhexoat, Zinn(II)-oleat und Zinn(II)-laurat sowie Zinn(IV)-Verbindungen wie Dibutylzinndiacetat, Dibutylzinndilaurat, Dibutylzinnmaleat, Dioctylzinndiacetat, Dibutylzinndi-2-ethylhexoat, Tributylzinnacetat, 1,1,3,3-Tetrabutyl-1,3-diacetoxydistannoxan oder z. B. Verbindungen vom Typ der Stannosiloxane wie Tetra-(dibutylacetoxystannoxy)silan.

Die Katalysatoren können einzeln oder in beliebigen Kombinationen eingesetzt werden, wobei die Kombination eines Zinnsalzes mit einem tert. Amin oft bevorzugt ist, da hierbei häufig Synergismus bezüglich der katalytischen Aktivität beobachtet wird.

Die Herstellung der Anlagerungsprodukte der Sulfonylisocyanate an die beispielhaft genannten Katalysatoren kann nach mehreren Varianten erfolgen. In allen diesen Varianten erfolgt jedoch die Bildung der Anlagerungsprodukte der Sulfonylisocyanate an die beispielshaft genannten Katalysatoren vorzugsweise innerhalb des Temperaturbereichs von 0 bis 80°C, insbesondere 10 bis 30°C unter Verwendung solcher Mengen der beiden Komponenten der Anlagerungsprodukte, daß auf jede Aminogruppe der tert. Amine bzw. jedes Carboxylatanion der Zinnsalze mindestens eine Isocyanato-sulfonyl-Gruppe der Sulfonylisocyanate entfällt. Die Verwendung eines Unterschusses an Sulfonylisocyanat ist, wie leicht einzusehen, wenig zweckmäßig, da hierdurch nur eine teilweise Blockierung der Katalysatoren erreicht würde.

Im Falle der Verwendung von tert. Aminen wird vorteilhaft wie folgt vorgegangen:.

Das tert. Amin wird in der Gesamtmenge der Polyisocyanat-Komponente aufgelöst, worauf sich die Zugabe des Sulfonylisocyanats unter Rühren anschließt. Dabei können sowohl das tert. Amin als auch das Sulfonylisocyanat als auch die Polyisocyanat-Komponente in einem inerten Lösungsmittel gelöst sein. Das

gegebenenfalls mitverwendete Lösungsmittel kann im Anschluß an die Umsetzung zwischen tert. Amin und Sulfonylisocyanat gewünschtenfalls destillativ entfernt werden. Die genannte Umsetzung kann selbstverständlich ebenso gut auch unter Verwendung nur eines Teils der Polyisocyanat-Komponente als Reaktionsmedium durchgeführt werden. In einem solchen Fall würde das Reaktionsgemisch im Anschluß an die Umsetzung (Bildung der Anlagerungsverbindung) mit der Restmenge der Polyisocyanat-Komponente abgemischt werden. Hierbei können selbstverständlich unterschiedliche Polyisocyanate der oben beispielhaft genannten Art zum Einsatz gelangen. In einer weniger bevorzugten Variante kann man auch das Sulfonylisocyanat in der Polyisocyanat-Komponente oder in einem Teil der Polyisocyanat-Komponente bzw. deren Lösung in einem inerten Lösungsmittel vorlegen und dann das tert. Amin in die so erhaltene Lösung einrühren. Hierbei fallen jedoch häufig die erfindungswesentlichen Anlagerungsverbindungen aus, während nach der bevorzugten, erstgenannten Verfahrensvariante die gleichen Addukte in Lösung bleiben.

In einer weiteren weniger bevorzugten Verfahrensvariante kann man die Anlagerungsverbindung z. B. in einem inerten Lösungsmittel durch Kombination der Einzelkomponenten herstellen und die so erhaltene Lösung bzw. Dispersion der Anlagerungsverbindung der Polyisocyanat-Komponente einverleiben. Auch gemäß dieser, weniger bevorzugten Verfahrensvariante entstehen häufig sedimentierende Dispersionen der Anlagerungsverbindungen, deren Sedimente jedoch durch einfaches Aufrühren leicht redispergierbar sind. Selbstverständlich sind stabile Lösungen der Anlagerungsverbindungen in der Polyisocyanat-Komponente gegenüber den Dispersionen bevorzugt. Die überraschende Beobachtung, daß bei der erstgenannten bevorzugten Herstellungsvariante derartige stabile Lösungen entstehen, kann evtl. damit erklärt werden, daß sich hierbei in situ Isocyanatgruppen der Polyisocyanat-Komponente an die Anlagerungsverbindung, die vermutlich als Betain vorliegt, anlagern und so die überraschende gute Löslichkeit der in situ hergestellten Anlagerungsverbindungen bewirken.

Im gegensatz zu den Anlagerungsverbindungen auf Basis der beispielhaft genannten tert. Amine sind die Anlagerungsverbindungen auf Basis der beispielhaft genannten Zinnverbindungen (unabhängig von der Art ihrer Herstellung) in organischen Medien und somit auch in der Polyisocyanat-Komponente einwandfrei löslich. Bei Verwendung von organischen Zinnverbindungen der beispielhaft genannten Art können somit nach allen oben beschriebenen Varianten mit gleich gutem Erfolg Polyisocyanate erhalten werden, in denen der blockierte Katalysator in gelöster Form vorliegt.

Bei allen Verfahrensvarianten kommen die Polyisocyanat-Komponente und die Einzelkomponenten der Anlagerungsverbindung vorzugsweise in solchen Mengen zum Einsatz, daß schließlich im Polyisocyanat, bezogen auf die gesamte Polyisocyanat-Komponente, 0,005 bis 0,3 Gew.-%, vorzugsweise 0,05 bis 0,25 Gew.-% an tertiärem Aminstickstoff bzw. 0,008 bis 8,0 Gew.-%, vorzugsweise 0,01 bis 1,0 Gew.-% an Sn-Atomen vorliegen.

Die erfindungswesentlichen Anlagerungsverbindungen zwischen tert. Aminen und Sulfonylisocyanaten stellen sehr wahrscheinlich Betaine der folgenden Struktur dar:

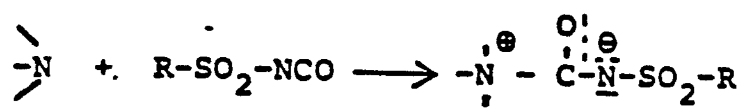

(vgl. DAS 1 100 618 und Z. Brzozowski u. W. Zackarewicz, Roczniki Chemii, 34, 1839 (1960)).

Die Struktur der erfindungswesentlichen Anlagerungsverbindungen der Zinnkatalysatoren ist nicht bekannt und kann evtl. mit den nachstehenden tautomeren Formeln beschrieben werden:

Zinn(II)-carboxylate:

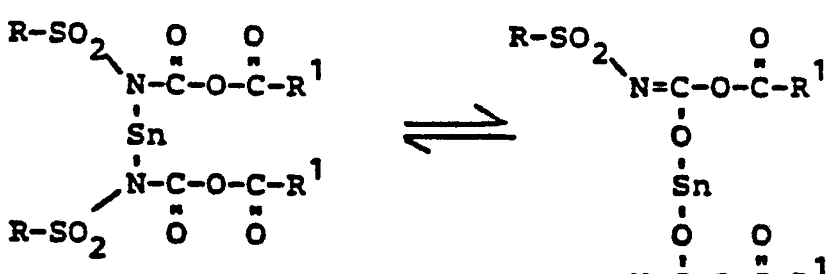

I

II

und z. B. bei Dialkylzinn(IV)-carboxylaten:

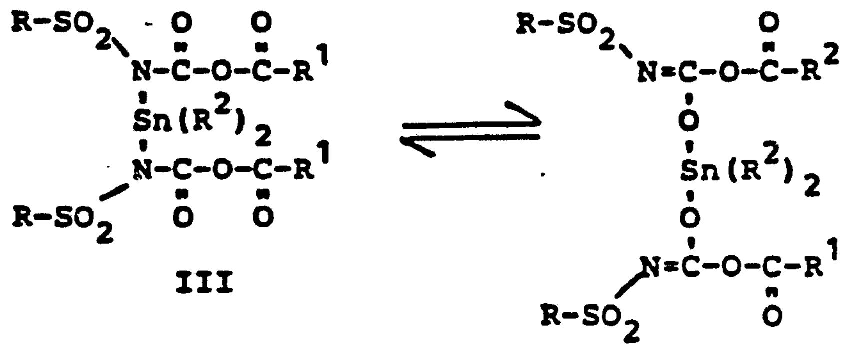

III

IV

In diesen Formeln steht R für den indifferenten Rest des Sulfonylisocyanats, $R^1$ für den indifferenten Rest des Carboxylatanions und $R^2$ für die indifferenten, direkt mit dem Zinnatom in homöopolarer Bindung verknüpften organischen Reste der Zinn(IV)-salze.

Die erfindungswesentlichen Anlagerungsverbindungen stellen 1 : 1-Addukte der Isocyanato-Sulfonyl-Gruppen an die tert. Aminogruppen bzw. die Carboxylatanionen dar. Die Anlagerungsverbindungen sind extrem hydrolyse-empfindlich und werden durch Zutritt von Wasser (z. B. durch Luftfeuchtigkeit) unter Freisetzung der Katalysatoren zerlegt. Vor dieser hydrolytischen Spaltung der erfindungswesentlichen Anlagerungsverbindungen sind diese gegenüber Isocyanatgruppen völlig inert und bewirken auch keinerlei die Lagerstabilität von organischen Polyisocyanaten beeinträchtigende Nebenreaktionen.

Die Herstellung der erfindungsgemäßen Verbandmaterialien erfolgt, indem man die oben beschriebenen flexiblen Trägermaterialien mit vorzugsweise 50 bis 300 Gew.-%, insbesondere 70 bis 200 Gew.-%, (bezogen auf Trägermaterial) des Gemisches aus Polyisocyanat und blockiertem Katalysator beschichtet und/oder imprägniert. Gegebenenfalls kann das Imprägnierungsmittel dabei mit einem inerten Lösungsmittel (z. B. Methylenchlorid) verdünnt sein, das nach dem Beschichtungsvorgang wieder abgezogen wird. Wegen der extremen Hydrolyseempfindlichkeit der erfindungsgemäß einzusetzenden Anlagerungsverbindungen muß unter völligem Ausschluß von Feuchtigkeit gearbeitet werden. Eventuell in den Ausgangsmaterialien (z. B. textiler Träger) oder im Luftraum noch vorhandene Feuchtigkeitsspuren können kompensiert werden, indem man einen Überschuß an Sulfonylisocyanat (bezogen auf die tert. Amin-Stickstoffatome bzw. die Zinnverbindungen) zwecks "Neutralisation" der Feuchtigkeit anwendet (vgl. US-PS-3 330 849 und US-PS-3 479 325).

Vorzugsweise erfolgt die Herstellung der erfindungsgemäßen Verbandmaterialien auch unter Sauerstoffausschluß, d.h. in einer trockenen Inertgas-Atmosphäre (vgl. DE-OS-3 033 659). Unmittelbar nach der Beschichtung bzw. Imprägnierung wird das Verbandsmaterial in der gewünschten Länge (in der Regel 2 bis 5 m) auf geeignete Spulen aufgerollt und in einer luft- und wasserdichten Folie (z. B. Polyethylen oder Aluminium) oder anderen völlig dichten Behältern versiegelt, wie es in DE-OS-2 357 931, DE-OS-2 651 089 und DE-OS-3 033 569 im Detail beschrieben wird.

Unmittelbar vor der Anwendung wird das Material aus der Verpackung entnommen und um den zu stützenden Körperteil gewickelt, der gegebenenfalls zuerst mit einem geeigneten Polster- bzw. Unterfütterungsmaterial (z. B. Polyestervlies) umgeben wurde. Die Aushärtung des Stützverbandes kann in verschiedener Weise erfolgen: So ist es z. B. möglich, das Verbandmaterial unmittelbar vor der Applikation in Wasser zu tauchen; man kann aber auch zunächst den trockenen Verband um den zu stützenden Körperteil wickeln und mit Wasser besprühen; schließlich ist es, wie schon erwähnt, auch möglich, den Verband durch bloßen Zutritt von Luftfeuchtigkeit auszuhärten. Je nach Anwendungsvariante ist die Menge an blockiertem Katalysator zu wählen, wobei die höheren Konzentrationen der oben angegebenen Bereiche für die Härtung durch Luftfeuchtigkeit in Frage kommen.

Vorteilhafterweise beginnt die Härtungsreaktion des erfindungsgemäßen Verbandmaterials nicht sofort beim Kontakt mit Wasser anzulaufen. Zunächst wird vielmehr der Katalysator ent-blockiert bzw. der vorhandene Überschuß an Sulfonylisocyanat hydrolysiert, so daß die eigentliche Vernetzungsreaktion zwischen Isocyanatgruppen und Wasser im wesentlichen erst nach einer gewissen "Inkubationszeit" anspringt, die ihrerseits wieder über die Konzentration an Katalysator bzw. Sulfonylisocyanat an Hand weniger Vorversuche gezielt eingestellt werden kann. Während dieser Inkubationszeit kann der Verband an den Körperteil des Patienten angelegt und modelliert werden.

Die nachfolgenden Beispiele erläutern die vorliegende Erfindung. Wenn nicht anders vermerkt, sind Mengenangaben als Gewichtsteile bzw. Gewichtsprozente zu verstehen.

**Polyisocyanat A** (Vergleich; gemäß DE-OS-2 651 089)

100 Teile eines technischen Polyphenyl-polymethylen-polyisocyanats, erhalten durch Phosgenierung eines Anilin-Formaldehyd-Kondensats ($\eta^{25}$ = 200 mPa.s; NCO-Gehalt: 31 %) werden mit 32,2 Teilen propoxyliertem Triethanolamin (OH-Zahl: 150) zu einem Prepolymer mit 20,2 % NCO-Gehalt und einer Viskosität $\eta^{25}$ = 20 000 mPa.s umgesetzt. Katalysatorgehalt: 0,31 % tert. Aminstickstoff.

**Polyisocyanat B** (erfindungsgemäß)

200 Teile des obigen technischen Phosgenierungsproduktes werden mit 52 Teilen Polypropylenglykol (OH-Zahl: 112) und 12,5 Teilen propoxyliertem Trimethylolpropan (OH-Zahl: 380) zu einem Prepolymer mit 19,5 % NCO-Gehalt umgesetzt. Danach rührt man unter Feuchtigkeitsausschluß 1,06 Teile Dibutylzinndilaurat und 1,32 Teile Tosylisocyanat ein. Auch nach 6-monatiger Lagerung unter Luftausschuß bei 25°C ist die Viskosität des Prepolymers praktisch unverändert, während es ohne Zusatz von Tosylisocyanat nach 6 Monaten völlig erstarrt ist. Katalysatorgehalt: 0,076 % Sn.

**Polyisocyanat C** (erfindungsgemäß)

100 Teile des obigen technischen Phosgenierungsproduktes werden mit 19,3 Teilen propoxyliertem Glycerin (OH-Zahl: 250) und 12,9 Teilen Polypropylenglykol (OH-Zahl: 56) zu einem Prepolymer mit 19,7 % NCO-Gehalt umgesetzt. Danach rührt man unter Feuchtigkeitsausschluß 1,3 Teile Bis-2-(dimethylamino)ethylether und 3,2 Teile Tosylisocyanat ein. Die Lagerstabilität unter Luftausschluß des Prepolymers entspricht jener unter B), während ohne Zusatz von Tosylisocyanat das Produkt bereits nach ca. 2 Monaten in einen Gelzustand übergegangen ist. Katalysatorgehalt: 0,17 % tert. Aminstickstoff.

Die Polyisocyanate wurden 1:1 mit $CH_2Cl_2$ verdünnt und bei 22°C und 55 % rel. Luftfeuchte auf Glas zu einem 0,2 mm dicken Film ausgegossen. Die Härtungszeiten (in Minuten) sind in der nachstehenden Tabelle angegeben:

| Polyisocyanat | Klebfreiheit | Grifftrockenheit |
|---|---|---|
| A | 10 | 26 |
| B | 20 | 56 |
| C | 10 | 30 |

Trockene Baumwoll-Mullbinden von 2 m Länge wurden unter Wasserausschluß mit den obigen Polyisocyanaten (6,0 g Binde/9,0 g Polyisocyanat), gelöst in $CH_2Cl_2$, imprägniert und das Lösungsmittel im Vakuum abgezogen. Die Binden wurden nach Eintauchen in Wasser um ein Rohr (Durchmesser 4,5 cm) gewickelt und aushärten gelassen. Die Härtungszeiten (in Minuten) sind in der nachstehenden Tabelle angegeben:

| Polyisocyanat | Klebfreiheit | Strukturfestigkeit |
|---|---|---|
| A | 7 | 10-12 |
| B | 10 | 15-17 |
| C | 9 | 10-12 |

**Polyisocyanat D** (erfindungsgemäß)

100 Teile des Polyisocyanats B werden zusätzlich mit 1,0 Teilen N,N-Dimethylcyclohexylamin und 4,6 Teilen Tosylisocyanat vermischt. Katalysatorgehalt: 0,076 % Sn; 0,11 % tert. Aminstickstoff. Die Lagerstabilität entspricht der von B, ohne Tosylisocyanat ist das Produkt nach 1 Monat erstarrt.

**Polyisocyanat E** (erfindungsgemäß)

100 Teile des Polyisocyanats B werden zusätzlich mit 0,6 Teilen 1,1,3,3-Tetrabutyl-1,3-diacetoxy-distannoxan, $[(C_4H_9)_2(CH_3COO)_2Sn]_2O$, und 0,8 Teilen Tosylisocyanat verrührt. Katalysatorgehalt: 0,31 % Sn. Die Lagerstabilität entspricht der von B; ohne Tosylisocyanat ist das Produkt nach 2 Monaten erstarrt.

Die Polyisocyanate A, D und E wurden nach dem zuvor angegebenen Verfahren auf Mullbinden aufgebracht und gewickelt und dann bei 22°C/55 % rel. Luftfeuchte aushärten gelassen. Es ergaben sich folgende

Härtungszeiten (in Minuten) zum Erreichen der Strukturfestigkeit:

Polyisocyanat A: 21 - 23
D: 24 - 26
E: 14 - 15

**Polyisocyanat F** (erfindungsgemäß)

100 Teile eines technischen Polyphenylpolymethylenpolyisocyanats, erhalten durch Phosgenierung eines Anilin-Formaldehyd-Kondensats ($\eta^{25°C}$ = 200 mPa.s, NCO-Gehalt: 31 %) werden mit 32 Teilen eines propoxylierten Trimethylolpropans (OH-Zahl: 150) zu einem Prepolymer mit 20,1 % NCO-Gehalt und einer Viskosität $\eta^{25°C}$ = 18000 mPa.s umgesetzt. Danach vermischt man mit 0,82 Teilen Trimethylsilyloxysulfonylisocyanat und anschließend 1,32 Teilen Dibutylzinndilaurat. Katalysatorgehalt.: 0,19 % Sn. Die Lagerstabilität ist größer als zwei Jahre ohne das Sulfonylisocyanat liegt sie bei einem Monat.

Polyisocyanat F wurde im Vergleich zu Polyisocyanat A nach dem zuvor angegebenen Verfahren auf Mullbinden von 4 m Länge aufgebracht (12 g Binde / 18 g Polyisocyanat) und nach entsprechender Wassertauchung gewickelt und aushärten gelassen. Polyisocyanat A ist nach 7 Minuten klebfrei und nach 9 Minuten ausgehärtet. Polyisocyanat F ist nach 9 Minuten klebfrei und nach 14 Minuten ausgehärtet.

**Patentansprüche**

1. Bei Zutritt von Feuchtigkeit härtendes Material für die Herstellung von Stützverbänden in der Human- oder Tiermedizin, auf Basis eines flexiblen Trägermaterials, welches mit einer Isocyanatgruppen aufweisenden Verbindung imprägniert und/oder beschichtet ist, dadurch gekennzeichnet, daß die Isocyanatgruppen aufweisende Verbindung ein Anlagerungsprodukt eines Sulfonylisocyanats an ein tertiäres Amin und/oder an eine Verbindung mit Zinn(II)- bzw. Zinn(IV)-carboxylat-Struktur enthält.

2. Material nach Anspruch 1, dadurch gekennzeichnet, daß die Isocyanatgruppen aufweisende Verbindung 0,005 bis 0,3 Gew.-%, bevorzugt 0,05 bis 0,25 Gew.-% an tertiären Stickstoffatomen als Anlagerungsprodukt enthält.

3. Material nach Anspruch 1, dadurch gekennzeichnet, daß die Isocyanatgruppen aufweisende Verbindung 0,008 bis 8 Gew.-%, bevorzugt 0,01 bis 1,0 Gew.-% an Zinnatomen als Anlagerungsprodukt enthält.

4. Material nach Anspruch 1 oder 2, dadurch gekennzeichnet, daß der mit Sulfonylisocyanat blockierte tertiäre Aminstickstoff chemisch in die Isocyanatgruppen aufweisende Verbindung eingebaut ist.

5. Material nach Ansprüchen 1 bis 3, dadurch gekennzeichnet, daß die Isocyanatgruppen aufweisende Verbindung das Anlagerungsprodukt in gelöster oder dispergierter Form enthält.

6. Material nach Ansprüchen 1 bis 5, dadurch gekennzeichnet, daß die Isocyanatgruppen aufweisende Verbindung ein Prepolymer mit einem mittleren Molekulargewicht von 400 bis 10 000 ist.

7. Material nach Ansprüchen 1 bis 6, dadurch gekennzeichnet, daß die Isocyanatgruppen aromatisch gebunden sind.

8. Material nach Ansprüchen 1 bis 7, dadurch gekennzeichnet, daß das Sulfonylisocyanat eine Verbindung mit mindestens einer aromatisch gebundenen Isocyanatosulfonyl-Gruppe darstellt.

9. Material nach Ansprüchen 1 bis 8, dadurch gekennzeichnet, daß das Sulfonylisocyanat im Überschuß gegenüber tertiärem Aminstickstoff und/oder Zinn vorliegt.

10. Material nach Ansprüchen 1 bis 9, dadurch gekennzeichnet, daß es in einem luft- und feuchtigkeitsundurchlässigen Behälter verpackt ist.

**Claims**

1. Material which hardens on access of moisture, for the production of support dressings in human medicine or veterinary medicine, which is based on a flexible carrier material impregnated and/or coated with a compound containing isocyanate groups, characterised in that the compound containing isocyanate groups contains an addition product of a sulphonyl isocyanate on a tertiary amine and/or on a compound with a tin(II) or tin(IV) carboxylate structure.

2. Material according to Claim 1, characterised in that the compound containing isocyanate groups contains 0.005 to 0.3 % by weight, preferably 0.05 to 0.25 % by weight, of tertiary nitrogen atoms as the addition product.

3. Material according to Claim 1, characterised in that the compound containing isocyanate groups contains 0.008 to 8 % by weight, preferably 0.01 to 1.0 % by weight, of tin atoms as the addition product.

4. Material according to Claim 1 or 2, characterised in that the tertiary amine nitrogen blocked with sulphonyl

isocyanate is incorporated chemically into the compound containing isocyanate groups.

5. Material according to Claims 1 to 3, characterised in that the compound containing isocyanate groups contains the addition product in dissolved or dispersed form.

6. Material according to Claims 1 to 5, characterised in that the compound containing isocyanate groups is a prepolymer with an average molecular weight of 400 to 10 000.

7. Material according to Claims 1 to 6, characterised in that the isocyanate groups are aromatically bonded.

8. Material according to Claims 1 to 7, characterised in that the sulphonyl isocyanate is a compound with at least one aromatically bonded isocyanatosulphonyl group.

9. Material according to Claims 1 to 8, characterised in that the sulphonyl isocyanate is present in excess compared with the tertiary amine nitrogen and/or tin.

10. Material according to Claims 1 to 9, characterised in that it is packaged in a container which is impermeable to air and moisture.

**Revendications**

1. Matériau durcissant à l'humidité pour la fabrication de bandages de soutien pour utilisation en médecine humaine ou vétérinaire, à base d'un matériau support flexible imprégné et/ou revêtu d'un composé présentant des groupes isocyanate, caractérisé en ce que le composé présentant des groupes isocyanate est un produit d'addition d'un sulfonylisocyanate sur une amine tertiaire et/ou sur un composé comportant une structure de carboxylate stanneux ou stannique.

2. Matériau selon la revendication 1, caractérisé en ce que le composant présentant des groupes isocyanate contient 0,005 à 0,3 % en poids, de préférence 0,05 à 0,25 % en poids d'atomes d'azote amino tertiaires.

3. Matériau selon la revendication 1, caractérisé en ce que le composé présentant des groupes isocyanate contient comme produit d'addition 0,008 à 8 % en poids, de préférence 0,01 à 1,0 % en poids d'atomes d'étain.

4. Matériau selon la revendication 1 ou 2, caractérisé en ce que l'azote amino tertiaire bloqué avec le sulfonylisocyanate est chimiquement incorporé dans le composé comportant les groupes isocyanate.

5. Matériau selon les revendications 1 à 3, caractérisé en ce que le composé comportant les groupes isocyanate contient le produit d'addition sous forme dissoute ou dispersée.

6. Matériau selon les revendications 1 à 5, caractérisé en ce que le composé comportant les groupes isocyanate est un prépolymère de poids moléculaire moyen de 400 à 10 000.

7. Matériau selon les revendications 1 à 8, caractérisé en ce que les groupes isocyanate sont liés à un ou plusieurs groupes aromatiques.

8. Matériau selon les revendications 1 à 7, caractérisé en ce que le sulfonylisocyanate représente un composé avec au moins un groupe isocyanato-sulfonyle lié à un groupe aromatique.

8. Matériau selon les revendications 1 à 8, caractérisé en ce que le sulfonylisocyanate se trouve en excès par rapport à l'azote amino tertiaire et/ou l'étain.

10. Matériau selon les revendications 1 à 8, caractérisé en ce qu'il est emballé dans un conteneur imperméable à l'air et à l'humidité.